Europäisches Patentamt

European Patent Office

Office européen des brevets

(19)

(11) Veröffentlichungsnummer: **0 088 900**
**B1**

# EUROPÄISCHE PATENTSCHRIFT

(12)

(45) Veröffentlichungstag der Patentschrift:
02.09.87

(21) Anmeldenummer: 83101489.9

(22) Anmeldetag: 16.02.83

(51) Int. Cl.⁴: **A 61 M 1/10,** A 61 M 1/14

(54) Vorrichtung zum Reinigen des Blutes von Stoffwechselprodukten.

(30) Priorität: 16.02.82 DE 3205449

(43) Veröffentlichungstag der Anmeldung:
21.09.83 Patentblatt 83/38

(45) Bekanntmachung des Hinweises auf die Patenterteilung:
02.09.87 Patentblatt 87/36

(84) Benannte Vertragsstaaten:
BE FR GB IT

(56) Entgegenhaltungen:
DE-A-2 619 324
DE-A-2 619 389
US-A-3 007 416
US-A-3 536 423
US-A-3 791 767
US-A-4 250 872

(73) Patentinhaber: Fresenius AG, Gluckensteinweg 5,
D-6380 Bad Homburg (DE)

(72) Erfinder: Polaschegg, Hans- Dietrich, Dr.,
Grünwiesenweg 9, D-6370 Oberursel 4 (DE)
Erfinder: Mathieu, Bernd, Dr., Am unteren
Galgenberg 19, D-6683 Spiesen (DE)

(74) Vertreter: Luderschmidt, Wolfgang, Dr. Dipl-
Chem., Görtz, Dr. Fuchs, Dr. Luderschmidt
Patentanwälte Sonnenberger Strasse 100
Postfach 26 26, D-6200 Wiesbaden (DE)

EP 0 088 900 B1

## Beschreibung

Die Erfindung betrifft eine Vorrichtung zum Reinigen des Blutes von Stoffwechselprodukten, insbesondere Single-Needle-Dialysevorrichtung, gemäß dem ersten Teil des Anspruch 1.

Die US-A- 37 91 767 beschreibt eine Vorrichtung der eingangs erwähnten Art, die eine Pumpanordnung in einem Single-Needle-Dialysesystem aufweist. Bei dieser Pumpanordnung wird das Blut in eine schlauchförmige, flexible Speicherkammer in einem ersten Schritt eingepumpt und aus dieser im nächsten Schritt abgepumpt. Diese flexible Speicherkammer ist in einem starren Gehäuse angeordnet und bildet in diesem einen Ringraum, in den durch Pumpen eine Flüssigkeit als Pumpfluid gefördert wird.

Die Pumpe gemäß US-A- 37 91 767 weist einen mit einem Balg verbundenen Kolben auf, der gegen die Kraft einer Feder bewegt wird. Da sich das Balginnenvolumen verändert und hierdurch ein Unterdruck erzeugt wird, erfolgt ein Ansaugen der Flüssigkeit aus dem Ringraum des Speichers. Das Blut wird dabei solange angesaugt, bis sich der Schlauch an die Speicherwand angelegt hat, ohne daß er jedoch erweitert worden wäre.

Das Blutvolumen ist durch die in der Pumpanordnung befindliche Flüssigkeitsmenge und das Innenvolumen des nicht elastisch erweiterbaren Schlauchs begrenzt.

Um das Blut aus dem Schlauch abzupumpen, wird der Solenoid, der den Balg bewegt, abgeschaltet. Durch die Rückschnellkraft der Feder wird der Balg anschließend zusammengepreßt, so daß die Pumpflüssigkeit in den Speicher zurück gepumpt wird.

Da dieses System volumenkonstant ist, ergeben sich einige schwerwiegende Nachteile.

Zunächst sind die auf das Blut einwirkenden Unter- und Überdrücke nicht beliebig einstellbar, da sie weitgehend von der Auslegung der einzelnen Pumpelemente festgelegt sind. So stellt die Feder infolge ihrer konstanten Federkraft grundsätzlich den Balg und somit die Pumpflüssigkeit mit konstantem Druck zurück, die wegen der Inkompressibilität diesen Druck an das Blut weitergibt. Da für den Solenoid ähnliche Betriebsbedingungen gelten, ist davon auszugehen, daß keine Drucksteuerung vorliegt. Dies führt dazu, daß das zu pumpende Flüssigkeitsvolumen relativ genau eingestellt werden muß, um die Pumpe effektiv auszunutzen.

Weiterhin fördert der Balg zwar die Pumpflüssigkeit zurück, erzeugt jedoch keinen steuerbaren Unterdruck im Pumpsystem, da das Blut ohne einen Strömungswiderstand in den Schlauch nachströmt und der Schlauch selbst aus seinem kollabiertem Zustand ohne Unterdruck in den expandierten, jedoch immer noch drucklosen Zustand, überführt wird.

Ein bedeutender Nachteil dieser Pumpanordnung besteht darin, daß bei Betriebsbeginn die Phasenlage zwischen dem Speicher einerseits und der Balgpumpe andererseits übereinstimmen muß, damit die Pumpanordnung überhaupt funktionieren kann. Eine solche Übereinstimmung läßt sich jedoch regelmäßig nicht oder nur schwer erzielen, mit der Folge, daß nur noch eine unvollständige Füllung der Speicherkammer mit Blut stattfindet. Dies gilt insbesondere bei der Verwendung eines inkompressiblen Mediums als Pumpfluid. Da sich weiterhin die Pumpverhältnisse während des Betriebs ändern können, beispielsweise die Phasenlage zwischen der Speicherkammer und der Pumpe verschoben wird, variiert in entsprechender Weise das Pumpergebnis, das regelmäßig nicht zufriedenstellend ist.

Diese bekannte Pumpanordnung weist also weder in der Anlaufphase noch während der normalen Betriebsphase ein völlig zufriedenstellendes Pumpergebnis auf, was darauf zurückzuführen ist, daß außer der Menge der Pumpflüssigkeit sämtliche Betriebsparameter praktisch konstant und somit nicht mehr steuerbar sind.

Die US-A- 38 11 800 beschreibt ein weitgehend ähnliches System, bei dem wiederum ein sich nach dem Zusammendrücken in die Ausgangslage zurückstellender, jedoch nicht erweiterbarer Schlauch als Speicherelement eingesetzt wird. Auf diesen Schlauch wirkt ein weiterer Schlauch ein, der mit einem hydraulischen Pumpfluid gefüllt ist. Durch eine Erweiterung dieses Schlauchs wird der mit Blut gefüllte Schlauch zusammengedrückt und das im Schlauch befindliche Blut abgepumpt. Der Blutschlauch wird anschließend beim Zurückziehen der Pumpflüssigkeit zurückgestellt und füllt sich somit bei entsprechender Steuerung der Klemmen, die den Blutschlauch abschließen und öffnen.

Es wird also mit einer hydraulisch angetriebenen Pumpe gepumpt, die innerhalb eines starren Gehäuses untergebracht ist. Das Volumen dieses Gehäuses ist dabei geringer als das Volumen der beiden Schläuche. So kommt es daher ständig zu einer Kollabierung eines der beiden Schläuche.

Derartige hydraulische Systeme weisen Probleme bei der Drucksteuerung auf, da der Kompressionsdruck nicht durch eine einfach zu gewährleistende Sicherheitsmaßnahme begrenzt werden kann.

Weiterhin kann die Blutansauggeschwindigkeit nicht geregelt werden, da sie durch die Rückschnellkraft des Blutschlauchs sowie die Eigenschaften des Pumpschlauches und des angeschlossenen Systems bestimmt ist. Eine weitere Regelung ist nicht möglich.

Die DE-A- 24 55 917 beschreibt einen extrakorporalen Kreislauf in Form eines Single-Dialysesystems, das wiederum eine Pumpe zum Umpumpen von Blut mit einem veränderlichen Innenvolumen aufweist. Zum Einsatz kommen die bereits als nachteilig beschriebenen Schlauchpumpen oder eine rohrförmige Membran und Ventile aufweisende Membranpumpen, die ein gesteuertes Einlaß- und Auslaßventil aufweisen. Derartige Pumpen sind als Ventrikelpumpen bekannt, die grundsätzlich selbstansaugend sind und den Blutfluß in eine Richtung steuern.

Ähnliche Pumpsysteme sind weiterhin aus der DE-A-21 49 026 und der DE-A- 26 19 324 bekannt, die als Blutpumpen eingesetzt werden sollen. Dabei handelt es sich um passiv betriebene Ausdehnungsgefäße, die sich regelmäßig selbsttätig füllen, ohne daß eine optimale Pumpleistung durch das Antriebselement erreicht werden kann.

Weiterhin ist ein solches balgartiges Ausdehnungsgefäß, das unmittelbar mit Blut beaufschlagt wird, in Medizinalmarkt/Acta Medicotechnica 26,(1978) Bd. 12, S. 381 - 383, beschrieben. Eine solche mechanisch angetriebene Balgpumpe hat den Nachteil, daß sie nicht per se den Überdruck oder Unterdruck einschränkt. Sie benötigt daher einen zusätzlichen, in das Blutzuführungssystem integrierten Druckaufnehmer, der wiederum die externe Pumpe, bzw. die Vorschubgeschwindigkeit steuert, mit der der Balg zusammen- oder auseinandergezogen wird.

Demgemäß kann also nur festgehalten werden, daß die vorstehend erläuterten Pumpsysteme entwerder einer volumenkonstanten Zwangssteuerung unterzogen sind oder aber eine mit der Pumpkammer gekoppelte Drucksteuerung aufweisen. Darüber hinaus können solche Blutpumpen zu Beginn ihrer Tätigkeit bei unbestimmter Phasenlage nur schwierig und mit einem speziellen Füllprogramm oder speziellen Einrichtungen hierfür betrieben werden.

Der Erfindung liegt daher die Aufgabe zugrunde, eine Vorrichtung der eingangs erwähnten Art zur Verfügung zu stellen, bei der die Pumpe in jeder Betriebsart sicher gefüllt und entleert werden kann, ohne daß die Hubkolbenpumpe über mit der Pumpe oder dem Blutzuführungssystem verbundene Druckaufnehmer geregelt werden muß.

Die Lösung der Aufgabe erfolgt durch die kennzeichnenden Merkmale des Anspruch 1.

Erfindungsgemäß erfolgt die Regelung der Hubkolbenpumpe unabhängig von einer mit der Pumpe in Verbindung stehenden Regeleinrichtung, d.h. die Pumpe regelt sich selbsttätig, wenn ein bestimmter an die Pumpe angelegter Uberdruck oder Ünterdruck erreicht wird. Dabei öffnen sich die Ventile, so daß der auf die Pumpe einwirkende Druck nicht mehr erhöht oder abgesenkt wird.

Eine derartige Betriebweise ist mit den eingangs erwähnten hydraulischen Pumpen nur schwierig durchzuführen, da die Pumpflüssigkeiten infolge ihres inkompressiblen Charakters regelmäßig in Pumpanordnungen mit konstantem Volumen untergebracht sind, d.h. Pumpe und Antrieb werden regelmäßig zwangsgesteuert.

Erfindungsgemäß kann jeder beliebige Über-und Unterdruck in der pneumatisch betriebenen Pumpe eingestellt werden, ohne daß diese beschädigt wird.

Weiterhin ist es vorteilhaft, daß die erfindungsgemäße Pumpanordnung zu Beginn ohne jegliche Schwierigkeit gefüllt werden kann, da sie von der Phasenlage unabhängig ist. Die Pumpe saugt nämlich Blut solange an, bis sich die elastische Membran an das starre Pumpgehäuse angelegt hat und dadurch der Druck abfällt. Wenn ein bestimmter Unterdruck erreicht ist, öffnet sich das Unterdruckventil und hält diesen Unterdruck solange konstant, bis die Pumpe den Umkehrpunkt erreicht. Ist dieser Umkehrpunkt erreicht, so schließt sich das Unterdruckventil und es wird die Pumpe entleert. Nach dem Entleeren steigt der Überdruck an und wird bei einem bestimmten Wert dadurch konstant gehalten, daß das Überdruckventil sich öffnet.

Die Pumpe arbeitet jedoch weiter und kehrt erst an ihrem anderen Umkehrpunkt zum Saugbetrieb zurück. An diesem Punkt hat sie nun die richtige Phasenlage erreicht.

Sie benötigt daher lediglich einen Zyklus, um zu der jeweils abgestrebten Phasenlage zu kommen, die einen optimalen Pumpbetrieb garantiert.

In einer besonders bevorzugten Ausführungsform ist das Innenvolumen der Pumpe und der Hubkolbenpumpe korreliert und somit das Verhältnis dieser Volumina begrenzt. Demzufolge unterliegt auch der Druck einer bestimmten Begrenzung in diesem pneumatisch betriebenen System, so daß u.U. auch der Ausfall der Druckbegrenzungsventile toleriert werden kann.

Weitere Einzelheiten, Vorteile und Ausführungsformen sind in der nachfolgenden Beschreibung unter Bezugnahme auf die Zeichnung erläutert.

Es zeigen

Fig. 1 schematisch eine Single-Needle-Dialyseanordnung mit dem erfindungsgemäßen Pumpsystem

Fig. 2 einen Längsschnitt durch eine erste Ausführungsform des erfindungsgemäßen Pumpspeichers

Fig. 3 einen Längsschnitt durch eine zweite Ausführungsform des erfindungsgemäßen Pumpspeichers

Fig 4. eine Druck/Volumen-Charakteristik der in Fig. 2 gezeigten Ausführungsform beim Befüllen

Fig. 5 eine Membranpumpe für das in Fig. 1 gezeigte Pumpsystem

Fig. 6 - 8 jeweils den Endabschnitt weiterer Ausführungsformen von Pumpspeichern im Längsschnitt, wobei eine weitere zylindrische Außenwand des Pumpspeichers skizziert angedeutet ist und

Fig. 9 eine schematische Darstellung eines kontinuierlich pumpenden Systems mit zwei Pumpspeichern.

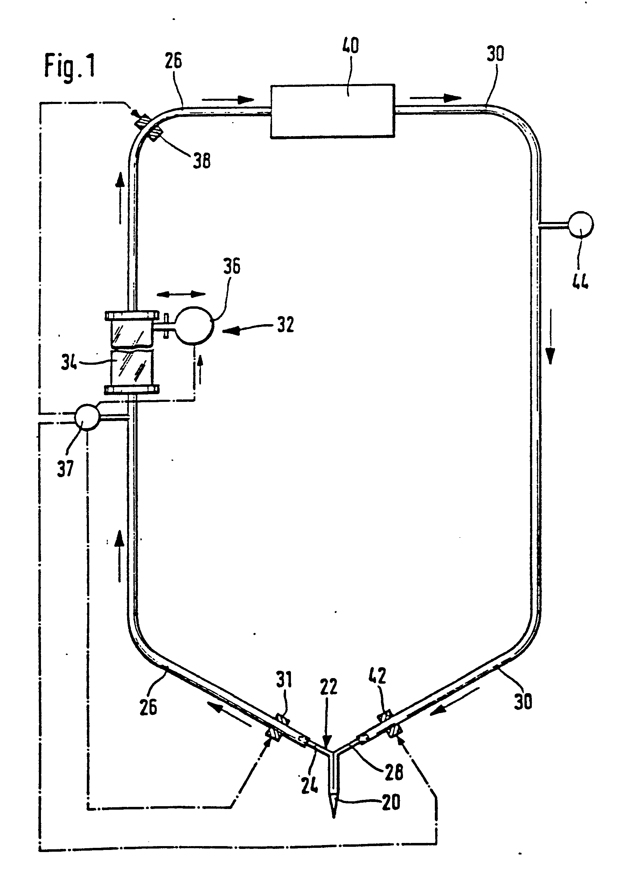

Gemäß Fig. 1 wird einem Patienten Blut über eine Hohlkanüle 20 Blut entnommen, die in ein Blutgefäß eingeschoben wird. Diese Hohlkanüle 20 weist an ihrem Ende einen y-förmigen Anschluß 22 auf, bei dem der eine Anschluß 24 mit der Arterienleitung 26 verbunden ist, während der andere Ast 28 mit der Venenleitung 30 verbunden ist. Die Hohlkanüle kann als Biflownadel ausgebildet sein, d.h. sie besteht aus zwei ineinander koaxial angeordneten Kanülen, bei denen die äußere Kanüle um wenige Millimeter zurückgesetzt ist, um einen Vermischungseffekt zu vermeiden. Andrerseits kann jedoch auch der geringe Vermischungseffekt bei der vorzugsweise eingesetzten y-förmigen Nadel 20 hingenommen werden.

In der Arterienleitung 26 ist im Anschluß an die y-förmige Hohlnadel 20 ein Absperrorgan 31 vorgesehen, mit der die Arterienleitung geöffnet und abgeschlossen werden kann. An dieses Absperrorgan 31 schließt sich ein Pumpsystem 32 an, das mit der Arterienleitung 26 in Verbindung steht und im wesentlichen die Pumpe 34 und die damit in Verbindung stehende Hubkolbenpumpe 36 aufweist. Stromab des Pumpsystems 32 ist in der Arterienleitung 26 ein weiteres Absperrorgan 38 angeordnet, das vorteilhafterweise entweder als Klemme oder aber als Rückschlagventil ausgebildet ist.

Stromab des Absperrorgans 38 ist die Arterienleitung 26 mit dem Dialysator 40 verbunden, in dem das zu reinigende Blut von den Stoffwechselprodukten und überschüssiger Flüssigkeit befreit wird. Dieser Dialysator 40 ist mit einer nicht gezeigten Dialyseanordnung verbunden, die die Dialyseflüssigkeit mit ebenfalls nicht gezeigten Zu- und Ableitungen durch den Dialysator 40 fördert.

An diesen Dialysator 40 schließtsich die Venenleitung 30 mit dem Leitungsast 28 an, der zur y-förmigen Kanüle 20 geführt ist. Vor der Kanüle 20 ist ein weiteres, als Klemme ausgebildetes Absperrorgan 42 vorgesehen, mit der die Venenleitung 30 geöffnet und geschlossen werden kann.

Weiterhin kann vorteilhafterweise in der Venenleitung 30 ein Drucksensor 44 angeordnet sein, der den Druck anzeigt, der sich in dem Leitungsabschnitt zwischen dem Absperrorgan 38 und der Klemme 42 aufbaut.

Die in Fig. 1 gezeigte erste Ausführungsform wird folgendermaßen betrieben:

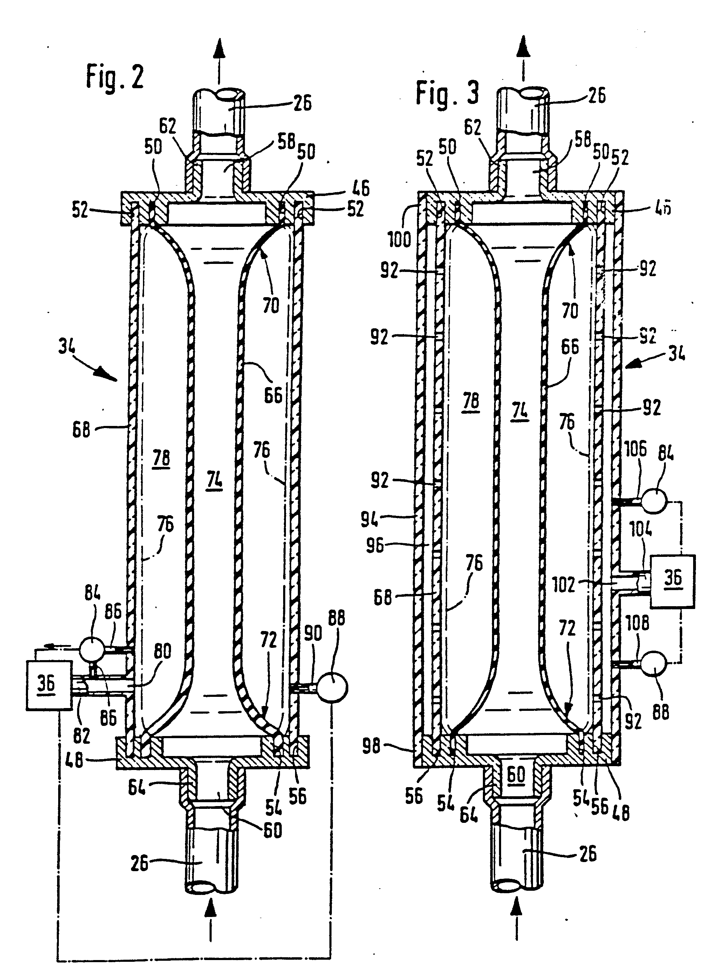

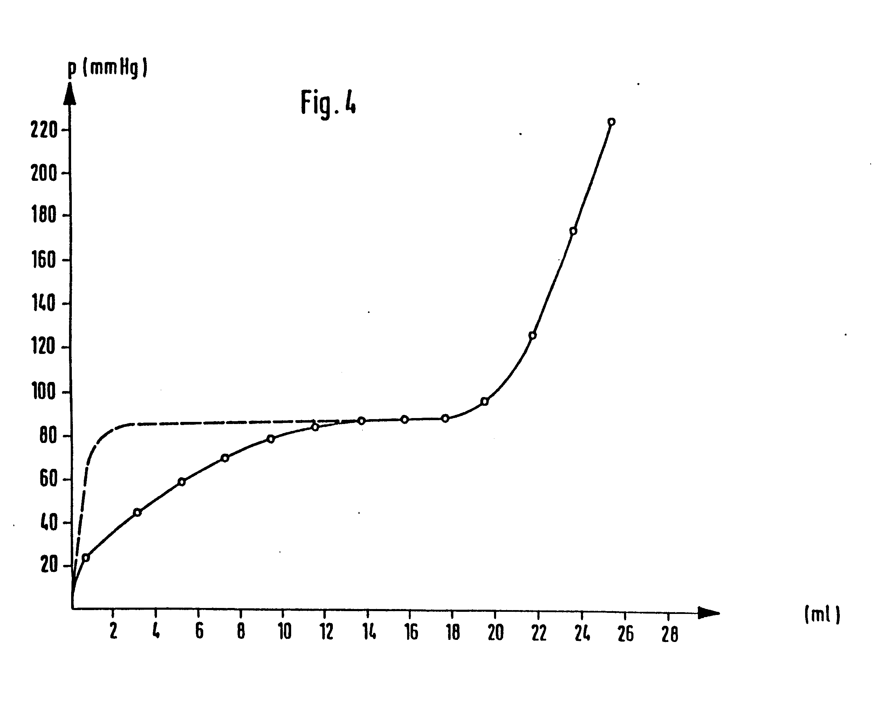

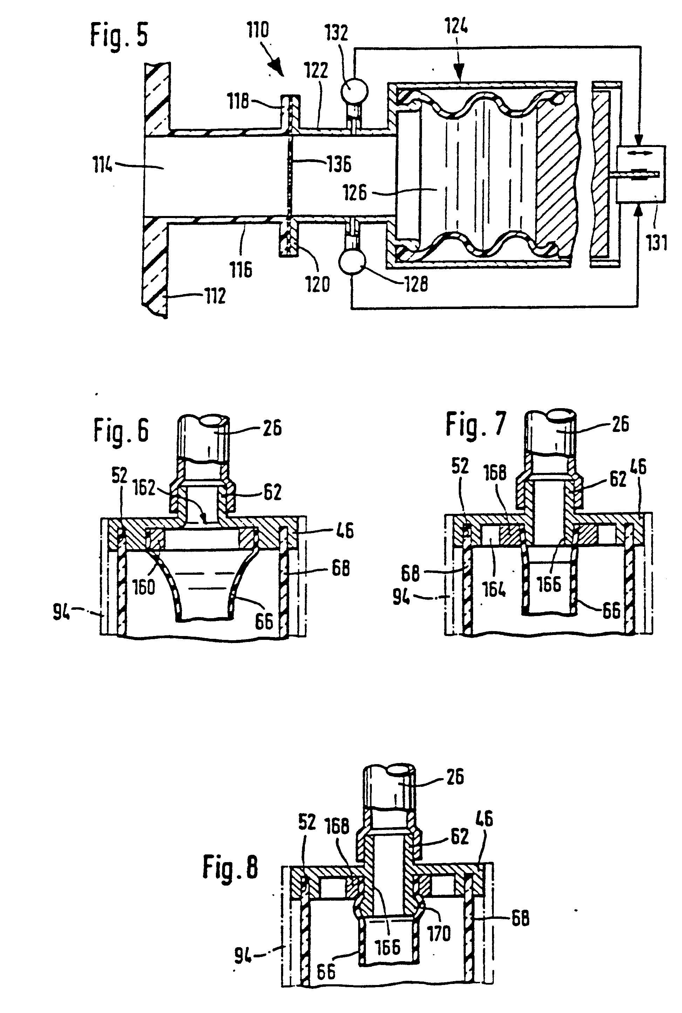

Das Blut wird über die Hohlkanüle 20 mittels des in Fig. 5 näher gezeigten und nachstehend beschriebenen Pumpsystems 32 angesaugt wobei das Organ 38 und die Klemme 42 geschlossen und das Absperrorgan 31 geöffnet sind. Über die Arterienleitung 26 wird das Blut in die Pumpe 34 mittels der Hubkolbenpumpe 36 gepumpt, wobei die anhand Fig. 2 nachstehend näher erläuterte schlauchförmig ausgebildete Pumpe 34 zunächst drucklos aufgefüllt wird. Anschließend erweitern sich unter sprunghaftem Druckanstieg die Außenwände, ähnlich dem Zustand, in dem ein Ballon aufgeblasen wird. Bereits eine geringe Menge zusätzliches Blut reicht aus, um diesen sprunghaften Druckanstieg in ein nur noch geringfügig ansteigendes Druckplateau umzuwandeln, d.h. daß bei der weiteren Einspeisung von Blut in die Pumpe 34 der Druck in der Pumpe 34 im wesentlichen nur noch geringfügig ansteigt oder sogar konstant bleibt, ohne zunächst wieder sprunghaft anzusteigen. Dieser Anstieg erfolgt dann, wenn ein Grenzzustand erreicht wird, bei dem sich die elastischen Eigenschaften der Speicherwand verändern. Diese Druckcharakteristik ist in der Fig. 4 gezeigt.

In Fig. 2 ist die erste Ausführungsform der erfindungsgemäßen Pumpe 34 im Längsschnitt gezeigt. Diese Pumpe 34 weist im wesentlichen kreisförmige Endplatten 46 und 48 auf, in die ringförmige Nuten 50, 52, 54 und 56 eingelassen sind. Beide Endplatten 46 und 48 weisen weiterhin eine Öffnung 58 und 60 auf, die jeweils von Anschlußstutzen 62 und 64 umgeben sind. Auf diese Anschlußstutzen 62 und 64 sind die Leitungen 26 aufgesteckt oder aufgeklebt.

Beide Endplatten 46 und 48 sind miteinander über einen erweiterbare Schlauch 66 und eine zylinderförmige Speicherwand 68 verbunden. Der Schlauch 66 ist in seinen Endbereichen 70 und 72 quergespannt und in die ringförmigen Nuten 50 und 54 der Endplatten 46 und 48 eingelassen und dort befestigt. In seinem mittleren Bereich ist er jedoch nicht quergespannt, sondern allenfalls vorteilhafterweise in Längsrichtung etwas vorgespannt. In die zweiten ringförmigen Nuten 52 und 56 der Endplatten 46 und 48 ist die Speicherwand 68 fest eingesetzt, was beispielsweise durch Kleben oder mechanisches Klemmen vorgenommen werden kann.

Wie aus Fig. 2 ersichtlich, ist der Innenraum 74 des Schlauchs 66 mit der Öffnung 58 und somit der Arterienleitung 26 in Verbindung und wird beim Betrieb mit Blut gefüllt. Hierdurch erfolgt eine Erweiterung der Wand des elastischen Schlauchs 66 solange, bis sich dieser an die Speicherwand 68 vollständig angelegt hat, wie dies durch die strichpunktierte Linie 76 angedeutet ist.

Um eine derartige elastische Erweiterung des Schlauchs 66 zu erreichen, muß natürlich auf die Außenwand des Schlauchs 66 ein Unterdruck ausgeübt werden,der in dem im wesentlichen ringförmigen Raum 78 angelegt wird, der zwischen dem Schlauch 66 und der Speicherwand 68 gebildet wird. Hierzu ist in der Speicherwand 68 wenigstens eine Öffnung 80 vorgesehen, die über eine Zuleitung 82 mit der Hubkolbenpumpe 36 in Verbindung steht, die in Fig. 5 näher dargestellt und erläutert ist. Diese Hubkolbenpumpe 36 pumpt über die Zuleitung 82 und die Öffnung 80 Luft aus bzw. in den ringförmigen Raum 78, was entweder zur Erweiterung oder zum Kollabieren des Schlauchs 66 führt.

Infolge seiner hochelastischen Eigenschaften kollabiert der Schlauch 66 in seinem in Fig. 2 gezeigten aufgespannten Zustand nicht vollständig, so daß zum völligen Abpumpen von Blut aus dem Innenraum 74 ein zusätzlicher Überdruck durch die Hubkolbenpumpe 36 erzeugt werden muß.

Für die Zuführung von Blut ist ein Unterdruck von etwa 150 und max. 500 mm Hg-Säule notwendig, der mit den Sensoren 37, 44, 84 oder 88 gemessen werden kann, wobei letztere mit dem ringförmigen Raum 78 und/oder der Zuleitung 82 über die Leitung 86 bzw. 90 in Verbindung stehen.

Als Material für den Schlauch 66 werden hochelastische Materialien eingesetzt, beispielsweise organische Polymere und deren Gemische, wie Polyurethane, Kautschuke, Silikonkautschuke, Latex, Gummi, regenerierter Gummi u.dgl. Vorzugsweise wird Latex eingesetzt, das ggf. übliche Zusatz- und die Elastizität verbessernde Mittel aufweist. Ein derartiger Schlauch 66 kann wenigstens um das Fünffache seines Lumens, insbesondere um das Zehnfache ausgedehnt werden, so daß er also - wenigstens in der bevorzugten Ausführungsform - das Zehnfache seines Innenvolumens zusätzlich aufnehmen kann.

Die Wandstärke eines derartigen Schlauches 66 liegt in einem Bereich von etwa 0,05 - 0,5, vorzugsweise 0,1 - 0,4 mm. Sie wird in Abhängigkeit von der gewünschten Druckcharakteristik und der Volumenerweiterung der einzusetzenden Pumpe 34 gewählt. Je dicker die Wandstärke des Schlauches 66 ist, desto höher ist der Anfangsdruck, der aufzuwenden ist, um zu dem über einen weiten Volumenbereich führenden Druckplateau zu kommen. Vorzugsweise wird die Wandstärke so gewählt, daß das Druckplateau oberhalb des in der Vene herrschenden Druckes, vorzugsweise über 20 mm Hg liegt. Dieser Druck muß also in der Venenleitung vorliegen, um überhaupt Blut in die Vene überführen zu können.

Das Schlauchvolumen und die Länge des Schlauches 66 richten sich nach dem Einsatzzweck sowie der Auslegung der Dialysevorrichtung. Üblicherweise wird der Durchmesser des Schlauchs 66 in einem Bereich von 5 - 15, vorzugsweise etwa 8 mm liegen, während die Länge des Schlauchs 10 - 20, vorzugsweise ca. 15 cm betragen kann. Es sind jedoch aber auch kürzere oder längere Abmessungen denkbar, sofern dies der Einsatzzweck erfordert. Wenn nur geringe Blutvolumina befördert werden, wird man eine entsprechend geringer dimensionierte Pumpe mit einem entsprechend kleineren Schlauch einsetzen als bei größeren Blutumsätzen.

Bei der ersten in Fig. 2 gezeigten Ausführungsform weist der Schlauch 66 eine von der einen Endplatte 48 zur anderen Endplatte 46 abnehmende Wandstärke auf. Vorzugsweise nimmt die Wandstärke über die Gesamtlänge der Pumpe 34 um bis zu 50 % ab. Ein derartig über seine Gesamtlänge im wesentlichen stetig in seiner Wanddicke abnehmender Schlauch kann dadurch erzeugt werden daß man einen Stab in eine flüssige Latexmasse eintaucht und diesen Stab anschließend aus der flüssigen Latexmasse herauszieht. Durch den flüssigen Zustand dieser Latexmasse fließt ein Teil der an dem Schlauch hängenden Masse herunter, so daß die Wandstärke des dadurch hergestellten Schlauchs von einem Schlauchende zum anderen Schlauchende stetig zunimmt. Hierdurch steigt das Druckplateau, das sich beim Aufblähen des Schlauches 66 ergibt, nach dem abrupten Anstieg, wie aus Fig. 4 ersichtlich, ebenfalls stetig an.

Eine derartige in Fig. 2 dargestellte Anordnung eines Schlauches 66 mit abnehmender Wanddicke zieht die Anordnung der Öffnung 80, Die mit der Hubkolbenpumpe 36 über die Zuleitung 82 in Verbindung steht, in der Nähe des Schlauchbereichs 72 nach sich, in dem der Schlauch 66 seine größte Wandstärke besitzt. Der Schlauch wird sich nämlich dort zuerst aufblähen, wo die Wandstärke des Schlauchs 66 am geringsten ist, d.h. im Bereich der Endplatte 46. Dabei legt sich dieser Endbereich an die Speicherwand 68 an und füllt sich unter Bildung einer Wellenfront in Richtung auf das die größte Wandstärke aufweisende Schlauchende.

In Fig. 3 ist eine weitere Ausführungsform gezeigt, wobei für gleiche Teile die gleichen in Fig. 2 verwendeten Bezugszeichen eingesetzt werden. In dieser Ausführungsform kommt vorzugsweise ein Schlauch 66 zum Einsatz, dessen Wandstärke über seine Gesamtlänge im wesentlichen gleich ist. Die Verwendung dieser Ausführungsform ist jedoch nicht auf einen Schlauch mit im wesentlichen gleicher Wandstärke beschränkt, so daß auch Schläuche mit abnehmender Wandstärke, wie in Fig. 2 gezeigt, eingesetzt werden können.

Die Ausführungsform gem. Fig. 3 unterscheidet sich von der in Fig. 2 gezeigten Ausführungsform im wesentlichen dadurch, daß die Speicherwand 68 eine Vielzahl von Öffnungen 92 aufweist, die beliebig über dem Umfang und die Länge dieser Speicherwand 68 vorgesehen sind. Über diese Öffnungen 92 kann die Luft, die von der Hubkolbenpumpe 36 ab- oder zugepumpt wird, entweder entweichen oder aber zufließen.

Weiterhin ist bei der zweiten Ausführungsform eine weitere Außenwand 94 vorgesehen, deren Durchmesser größer ist als der Durchmesser der Speicherwand 68 und die im wesentlichen die gleiche Form wie die Speicherwand 68 besitzt. Somit wird bei einer Zylinderform der beiden Wände 68 und 94 ein ringförmiger Raum 96 zwischen diesen beiden Wänden gebildet, der über die Öffnungen 92 mit dem ringförmigen Raum 78 in Verbindung steht. Die Außenwand 94 ist jeweils in ihrem Endbereich 98 und 100 mit den Endplatten 46 und 48 fest verbunden, wobei die gesamte Anordnung bis auf die in der Außenwand 94 vorgesehene Öffnung 102 als gas dicht anzusehen ist. Diese Öffnung 102 kann an beliebiger Stelle in der Außenwand 94 vorliegen, da sich der Schlauch 66 nur an die Speicherwand 68 anlegen kann und durch die Vielzahl der Öffnungen 92 sichergestellt ist, daß der Schlauch 66 beim Abpumpen der Luft nicht die Abströmöffnung zusetzt und dadurch ein weiteres Abpumpen von Luft verhindert.

Eine solche Ausführungsform ist insbesondere bei einem Schlauch mit gleichbleibender Wandstärke zweckdienlich, da dieser in aller Regel keine von einem Schlauchende zum anderen Schlauchende ablaufende Wellenbewegung mitmacht, sondern sich beim Füllen mit Blut an einer beliebigen Stelle erweitern kann. Durch diese Anordnung wird sichergestellt, daß auch hier eine gleichmäßige Füllung des Schlauches 66 bis zum Anlegen an die Speicherwand 68 erfolgt, wobei sich der Schlauch 66 über seine gesamte Länge an die Speicherwand 68 anlegen kann.

Die Öffnung 102 ist wiederum über eine Leitung 104 mit der Hubkolbenpumpe 36 in Verbindung. Weiterhin sind ebenfalls wie in der ersten Ausführungsform Sensoren 84 und 88 vorgesehen, die über Leitungen 106 und 108 und entsprechende Öffnungen in der Außenwand 94 mit dem ringförmigen Raum 96 in Verbindung stehen.

Zweckmäßigerweise werden die Abmessungen des Schlauchs 66 so gewählt, daß der Raum 74 etwa 50 - 80 ml beträgt, während der elastische Schlauch 66 im drucklosen Zustand etwa 10 - 15 ml faßt.

Hinzuzufügen ist, daß als Material für den elastischen Schlauch 66 alle wasserundurchlässigen Materialien in Frage kommen, die eine entsprechend große Bruchdehnung ( > 200 %) aufweisen, wozu die vorstehend genannten Materialien gehören.

Als Material für die Speicherwand 68 und die Außenwand 94 kommen Materialien in Frage, die im wesentlichen starr sind. So sind beispielsweise Polymerisate, wie Polyethylen, Acrylglas, PVC u. dgl. einsetzbar.

Als Fluid für die ringförmigen Räume 78 und 96 setzt man üblicherweise Luft ein.

Wenn bei den vorstehend geschilderten Ausführungsformen Blut in den Schlauch 66 durch Erzeugung von Unterdruck mittels der Hubkolbenpumpe 36 eingepumpt wird, steigt der Druck anfangs nur geringfügig an, da der Schlauch 66 sich unter geringer Dehnung füllt und dabei das Volumen nur geringfügig zunimmt. Danach bläht sich der Schlauch 66 zunächst an der Stelle auf, Die die geringste Wandstärke besitzt. Er legt sich dabei an die Speicherwand 68 solange an, bis die Luft aus dem gesamten Raum 78 verdrängt ist. Während bei der ersten Ausführungsform mit der abnehmenden Wandstärke des Schlauchs 66 die Dehnungsfronten sich in axialer Richtung ausbreiten und der aufzuwendende Unterdruck im wesentlichen gleich bleibt, verändert sich dieser stetig mit dem gemäß der zweiten Ausführungsform eingesetzten Schlauch 66, der eine im wesentlichen gleiche Wandstärke besitzt.

Wenn die Luft vollständig aus dem Raum 78 verdrängt ist, so daß der Schlauch 66 entsprechend der Linie 76 an der Speicherwand 68 anliegt, steigt der Druck sehr rasch an, da sich der Schlauch 66 nicht mehr verformen läßt.

In Fig. 4 ist die Druck/Volumen-Charakteristik eines Beispiels des ersten Druckspeichers in durchgezogener Linie gezeigt, während in gestrichelter Linie das Idealverhalten eines Druckspeichers mit gleichbleibender Wandstärke dargestellt ist. Das Diagramm bezieht sich natürlich auf das Blutvolumen, das bereits einem bei Normaldruck gefüllten Schlauch 66 zugeführt wird. Der gemäß der ersten Ausführungsform eingesetzte Schlauch ist durch Tauchen eines Stabes in ein Latexbad und langsames Herausziehen dieses Stabes hergestellt worden, was aus der stetig ansteigenden Druckcharakteristik in Abhängigkeit von zugeführten Blutvolumen zu ersehen ist.

Da sich im elastischen Schlauch 66 sowohl die Druck- als auch die Volumenverhältnisse verändern, können beide Parameter zur Steuerung der Pumpe 34 unabhängig voneinander oder zusammen eingesetzt werden.

In Fig. 5 ist eine spezielle Pumpanordnung mit 110 bezeichnet, die sowohl für die erste als auch die zweite Ausführungsform einsetzbar ist. Dabei ist die Wand 112, die die der Speicherwand 68 bzw. der Außenwand 94 entspricht, mit einer Öffnung 114 durchsetzt, an die sich auf der Innenseite der Wand 112 der ringförmige Raum 78 bzw. 96 anschließt. Auf der Außenseite der Wand 112 steht die Öffnung 114 mit der Leitung 116 in Verbindung, die der Leitung 82 bzw. 104 entspricht.

Diese Leitung 116 weist an ihrem anderen Ende ein Paßstück 118, beispielsweise einen Flansch oder einen Luer-Konus auf, mit dem die Pumpe 34 mit einem weiteren Paßstück 120 gasdicht verbunden werden kann. An dieses weitere Paßstück schließt sich die Leitung 122 an, die mit der Hubkonbenpumpe 124 verbunden ist.

Bevorzugt ist eine Hubkolbenpumpe 124, deren Hubvolumen größer ist als das Speichervolumen der Pumpe 34. Besonders bevorzugt ist eine Hubkolbenpumpe 124, deren Volumen bis zu 20 % größer ist als das Speichervolumen der Pumpe 34.

Mittels dieser Hubkolbenpumpe 124 wird die Luft aus dem Innen bereich der Pumpe 34 abgesaugt und in den Balg 126 überführt.

Bei der Zwangssteuerung, bei der die Hubkolbenpumpe zwischen einem oberen und unteren Totpunkt, d.h. einem Höchst- und Tiefstdruckwert pendelt, sind die Sensoren 84 und 88 nicht zwangsläufig notwendig. Setzt man nämlich eine Hubkolbenpumpe 124 mit konstantem Fördervolumen und konstanter, zuvor bestimmter Pumpgeschwindigkeit ein, so ist in der Leitung 122 ein Unterdruckventil 128 vorgesehen das sich öffnet, wenn ein bestimmter Unterdruck in dieser Leitung erreicht ist. Vorzugsweise wird man dieses Unterdruckventil 128 so einstellen, daß dieses umschaltet, wenn sich der Schlauch 66 vollständig mit Blut angefüllt hat und sich an die Speicherwand 68 angelegt hat. Die Hubkolbenpumpe 124 schaltet dann selbsttätig am Totpunkt in ihrer Pumpfunktion um und schaltet zugleich an diesem Punkt die Klemmen 31, 38 und 42.

Es erfolgt darauf das Abpumpen von Blut aus dem Schlauch 66 entweder mittels der Hubkolbenpumpe 124 selbst oder aber durch Belüften der Leitung 122 durch ein nicht gezeigtes Ventil, wobei sich der Schlauch 66 infolge seiner elastischen Eigenschaften selbst entlädt.

Ist der Schlauch 66 völlig zusammengedrückt, so steigt der Druck in der Außenkammer stark an, sofern der Balg 126 weiterhin komprimiert, was der Fall ist, wenn das Hubvolumen der Pumpe größer ist als das Speichervolumen der Pümpe 34. In diesem Fall ist ein Überdruckventil 132 in der Leitung 122 vorgesehen, über das die überschüssige Luft entweichen kann, so daß ein bestimmter Höchstdruckwert im System nicht überschritten wird. Am unteren Totpunkt der Hubkolbenpumpe 124 wird dann die Klemme 38 sowie die Venenklemme 42 geschlossen und die Arterienklemme 31 geöffnet.

Aus der vorstehenden Ausführung ist ersichtlich, daß kein Sensor zum Umsteuern der Hubkolbenpumpe benötigt wird. Dabei kann im Fehlerfall d.h. bei Versagen einer Klemme oder beim Verstopfen der Leitungen kein Druck im Blutsystem auftreten, der größer ist als der durch das Überdruckventil 132 in der Pumpleistung bestimmte Maximaldruck. Da die Hubkolbenpumpe 124, sofern deren Hubvolumen größer ist als das Füllvolumen der Pumpe 34, immer mit einem Überschuß an Luft arbeitet, wird auch bei einer Undichtigkeit in der Pumpleitung 122 zwischen Balg 126 und dem Ausdehnungsraum 78 immer das volle Füllvolumen des Ausdehnungsraums 78 gepumpt. Auf diese Weise erreicht man ein Pumpvolumen, das pro Hub dem Füllvolumen der Pumpe 34 entspricht. Aus der Zahl der Hübe, die man mittels eines nicht gezeigten Zählgeräts bestimmen kann, läßt sich direkt durch Multiplikation mit dem Pumpvolumen die durchgesetzte Menge bestimmen und anzeigen sowie aus derem zeitlichen Differential auch die Pumprate.

Bei der gesamten Anordnung ist es wenigstens nützlich festzustellen, ob eine Unterbrechung, beispielsweise ein Verstopfen oder Lösen der Leitung, an irgendeiner Stelle aufgetreten ist. Dies kann einerseits durch Überprüfung des Druckes in der Venen- oder Arterienleitung mittels der Drucksensoren 37 oder 44 erfolgen. Dieser Druck wird nämlich Schwankungen unterliegen, die aufgrund des Strömungswiderstandes der Leitung 26 bzw. 30 und der Nadel 20 unter Förderung von Blut in den entsprechenden Leitungen 26 und 30 auftreten. Diese Drucksensoren 37 und 44 sind mit einer nicht gezeigten zeitgesteuerten Überwachungseinrichtung in Verbindung, die bei einem unerwünschten Druckwert die gesamte Anlage ausschaltet.

Eine entsprechende Überwachungseinrichtung 131 kann jedoch auch bei dem gemäß Fig. 5 gezeigten Pumpsystem angeordnet sein. Diese Überwachungseinrichtung ist sowohl mit den Ventilen 128 und 132 als auch mit der Hubkolbenpumpe 124 verbunden. Dabei ersetzt diese Überwachungseinrichtung eine nicht gezeigte Einrichtung, die das Öffnen der Ventile 128 und 132 anzeigen kann. Ist beispielsweise eine der Leitungen 26 oder 30 blockiert, so wird der Ausdehnungsraum 78 nur mit einem bestimmten Blutvolumen gefüllt sein und sich ansonsten jedoch inkompressibel verhalten, da ja aufgrund der Blockierung Blut weder zu- noch abgeführt werden kann. Infolgedessen wird sich das Über- bzw. Unterdruckventil 128 bzw. 132 nicht bei dem vorgesehenen Bruchteil der Hubbewegung des Balges 126 bzw. nicht zum dafür vorgesehenen Zeitintervall in Abhängigkeit vom Zeitpunkt des Umschaltvorganges öffnen, sondern schon weit früher. In einem solchen Fall schaltet die Überwachungseinrichtung 131 die gesamte Anlage ab.

In einer weiteren Ausführungsform können die Drucksensoren 37 und 44 zu einem einzigen Drucksensor zusammengefaßt sein, der vorteilhafterweise in der Nähe des Y-Stücks 22 zwischen den Klemmen 31 und 42 liegt. Dieser Sensor kann wiederum mit der Überwachungseinrichtung 131 verbunden sein, wobei man in der Ansaugphase den dadurch hervorgerufenen negativen Druck ablesen kann, andererseits in der Druckphase den Überdruck bestimmen kann.

Die vorstehend erläuterte Anordnung läßt weiterhin die folgende Überwachungsautomatik für das Einregulieren der Pumpengeschwindigkeit zu: Ist die Ergiebigkeit des Shunts oder der Nadel 20 nicht groß genug im Vergleich zur eingestellten Pumpgeschwindigkeit, so wird bei der Expansion des Balges 126 ein Unterdruck auftreten, der wesentlich über einem für die Kombination aus Schlauchsystem und Nadel charakteristischen Wert liegt. Dies kann durch einen Drucksensor, beispielsweise den Sensor 37 oder 84 detektiert werden. Dieser Wert kann jedoch aber auch bei Vorhandensein eines Unterdruckventiles 128 durch den Öffnungszeitpunkt dieses Ventiles detektiert werden. Vorteilhafterweise kann man schließlich aber auch auf eine Detektion dieses Zustandes völlig verzichten, wobei man das Unterdruckventil bzw. den Umsteuerungssensor auf einen Wert stellt, der aufgrund der Daten des Schlauchsystems und der Nadel berechnet bzw. zuvor empirisch bestimmt werden kann. Ist die Ergiebigkeit des Shunts groß genug, so wird der Unterdruck diesen Wert erst überschreiten, wenn der Ausdehnungsraum 78 vollgefüllt ist. Ist dies nicht der Fall, so wird der Unterdruck diesen Wert bereits vorher überschreiten, wobei das Unterdruckventil 128 geöffnet und dadurch das Pumpvolumen und die Pumprate beschränkt werden. Somit erhält man eine Pumpvorrichtung, die mit einfachen Mitteln verfahrenstechnisch sicher ist, wobei die Pump geschwindigkeit optimal der Leistungsfähigkeit des Shunts angebracht ist. In diesem Fall wird man die Balgpumpe 124 mit hoher Geschwindigkeit arbeiten lassen und auf eine vollstandige Füllung des Ausdehnungsraums 78 verzichten. Ein Ablesen der geförderten Blutmenge aus der Zahl der Pumphübe ist dann allerdings nicht mehr möglich.

Im übrigen ist zu bemerken, daß die Anordnung eines Drucksensors in der Blutleitung wesentlich kritischer ist als im Pumpensystem selbst. An der Pumpleitung 122 kann der Drucksensor fest angeschlossen werden, wobei die Gefahr von Undichtigkeiten im wesentlichen vernachlässigbar ist. Im übrigen kann der Sensor mit Umgebungsluft beaufschlagt werden, ohne daß hierdurch Kontaminationsgefahren entstehen. Insofern kann natürlich ein ungeschützter Sensor verwendet werden.

Um sicherzustlllen, daß bei einem Platzen des Schlauchs 66 kein Blut in die Hubkolbenpumpe 36 dringt, weist in einer bevorzugten Ausführungsform jede von der Wand 68 und 94 abzweigende Leitung eine hydrophobierte Membran 136 auf, wie dies in Fig. 5 dargestellt ist. Eine derartige hydrophobierte Membran, beispielsweise auf der Basis von PTFE mit einer Dicke von 10 - 50 µm und einem Porendurchmesser von unterhalb 1 µm, hält das Blut bei den verwendeten Drücken von einem Ausströmen aus der Pumpe 34 zurück gestatttet jedoch den ungehinderten Durchtritt von Luft.

Über eine derartige Drucksteuerung läßt sich natürlich die in der Pumpe 34 gespeicherte Blutmenge beliebig feststellen, sofern die eingesetzten Sensoren vorher entsprechend justiert und geeicht wurden. Weiterhin kann hier durch eine bestimmte Blutmenge zyklisch durch den Dialysator 40 gepumpt werden, wobei die Pumpe 34 nicht vollgepumpt werden muß.

Das in Fig. 1 gezeigte Absperrorgan 38 kann wahlweise einen konstanten Öffnungsquerschnitt oder aber auch einen variablen Öffnungsquerschnitt aufweisen, was von der durch die Klemmenbetätigung durchzulassenden Blutmenge abhängt. Bei im wesentlichen konstanten Druckplateau wird man ein Absperrorgan 38 mit konstantem Öffnungsquerschnitt einsetzen, um gleiche Förder- und Druckverhältnisse in der Venenleitung 30 herzustellen. Andererseits wird man bei einem ansteigenden Druckplateau, also bei über die Öffnungszeit der Absperrorgane 38 variierenden Druck -und Förderverhältnissen eine Klemme mit variierbarem Öffnungsquerschnitt einsetzen, um die durch die Venenleitung 30 geförderten Mengen im wesentlichen konstant zu halten. Dieser Öffnungsquerschnitt wird durch eine nicht gezeigte Steuerungseinrichtung, die druck- oder volumengesteuert sein kann, variiert.

Eine derartige Anordnung hat den Vorteil, daß der an dem Dialysator anliegende Druck nicht identisch ist mit dem Pumpdruck in der Pumpe 34, so daß der Dialysator mit erheblich niedrigeren, beliebig gegenüber dem Speicherdruck einstellbaren Drücken betrieben werden kann.

Die Pumpe 34 kann natürlich auch hinter dem Dialysator 40, d.h. in der Venenleitung 30 angeordnet sein, wobein natürlich das Absperrorgan 38 entfällt.

Weiterhin kann auch stromab des Dialysators eine zusätzliche Klemme angeordnet sein, die vorzugsweise einen variierbaren Öffnungsquerschnitt besitzt. In Verbindung mit dem Absperrorgan 38 kann somit in diesem Zweig, der den Dialysator umfaßt, ein beliebiger unterhalb dem sonstigen Betriebsdruck liegender Druck eingestellt werden.

In Fig. 6 - 8 sind weitere Ausführungsformen der erfindungsgemäßen Pumpe 34 gezeigt, in denen speziell die Befestigung des aufblähbaren Schlauchs 66 an den Endplatten 46 und 48 erläutert ist. Gemäß Fig. 6 ist der Innenschlauch 66 an der Endplatte 46 mit Hilfe eines Klemmrings 160 befestigt, der vorzugsweise eine konusförmige Gestalt zum leichteren Einschieben dieser Anordnung in die Ausnehmung 162 der Endplatte 46 aufweist. Der Durchmesser des Klemmrings 160 ist dabei so bemessen, daß der aufgeweitete Schlauch 66 sicher in der Ausnehmung 162 gehalten wird.

Beim Einsetzen wird zunächst der Schlauch 66 geweitet und über den Klemmring 160 gezogen. Anschließend wird diese Anordnung in der Endplatte 46 festgeklemmt. Die Speicherwand 68 läßt sich dann in die entsprechende ringförmige Nut 52 einsetzen. Danach wird der Schlauch 66 durch die Speicherwand 68 durchgezogen und mit einem entsprechenden Klemmring in die Endplatte 48 eingesetzt.

In Fig. 7 ist eine weitere Befestigungsart des Innenschlauchs 66 gezeigt. Dabei weist die Platte 46 auf ihrer Innenseite eine weite ringförmige Nut 164 auf, während die ringförmige Nut 52 für die Speicherwand 68 beibehalten wird. Auf den durch die ringförmige Nut 164 in der Endplatte 46 gebildeten Rohransatz 166 wird der Schlauch 66 mit Hilfe eines Klemmrings 168 befestigt, wobei die aus dem Schlauch 66 und dem Klemmring 168 bestehende Anordnung auf diesen Rohransatz 166 formschlüssig aufgeschoben wird.

In Fig. 8 ist eine Abwandlung der in Fig. 7 erläuterten Ausführungsform dargestellt. Dabei weist der Rohransatz 166 an seinem unteren Ende einen olivenförmigen Wulst 170 auf, dessen Außenumfang etwas großer ist als der Innenumfang des Klemmrings 168. Dieser Klemmring 168 ist jedoch so elastisch, daß er zusammen mit dem Schlauch 66 über den Wulst 170 geschoben werden kann und durch den Wulst 170 fest in Stellung gehalten wird.

Die in Fig. 6 - 8 gezeigte Pumpe 34 kann natürlich auch die in Fig. 3 gezeigte Außenwand 94 aufweisen, was durch die strichpunktierten Linien angedeutet ist.

Weiterhin ist es bevorzugt, daß der Schlauch 66 axial in der Pumpe 34 vorliegt. Vorteilhafterweise wird der Schlauch 66 um mindestens etwa 10 % seiner Gesamtlänge in Axialrichtung gedehnt, um ein Zusammenfallen zu verhindern. In Verbindung mit der vorstehend beschriebenen Querdehnung im Endbereich wird dadurch ein voll aufgespannter Schlauch 66 in der Pumpe 34 erhalten. Hierdurch erreicht man eine bessere Aufblähbarkeit des Schlauchs 66 im Betrieb. Zugleich wird ein Zusammenkleben der Schlauchwände durch fortschreitende Vulkaniesierung im Lagerzustand vermieden.

Die erfindungsgemäße Pumpe 34 läßt sich ohne Probleme sterilisieren und wird bereits in Verbindung mit dem Schlauchsystem einsatzbereit dem Patienten zur Verfügung gestellt. Bettseitig muß lediglich noch die Hubkolbenpumpe an die Pumpe 34 angeschlossen werden.

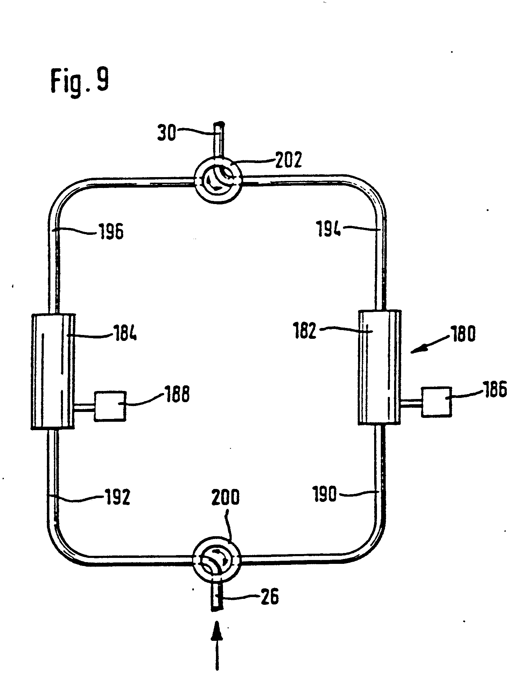

In Fig. 9 ist schematisch ein Pumpensystem 180 gezeigt, das für eine übliche Vorrichtung zur Hämodialyse eingesetzt werden kann, die mit zwei Nadeln arbeitet. In einem solchen System wird kontinuierlich Blut aus der Arterie über die Leitung 26 abgepumpt und über die Leitung 30 wieder der Vene zugeführt. Da jedoch die Pumpe 34 nicht kontinuierlich beladen und zugleich entladen werden kann, sind entsprechend der in Fig. 9 gezeigten Ausführungsform wenigstens zwei Pumpen 182 und 184 vorgesehen, die durch die Pumpen 186 und 188 angetrieben werden.

Dabei können die Pumpen 182 und 184 den Pumpen 34 gemäß Fig. 2 oder Fig. 3 entsprechen, während die zum Einsatz kommenden Pumpen 186 und 188 in Form der vorstehend beschriebenen Hubkolbenpumpen 36 und 124 vorliegen können.

Die Zuführungsleitung 26 verzweigt sich stromauf der beiden Pumpen 182 und 184 in wenigstens zwei Leitungsäste 190 und 192 und führt stromab der Pumpen 182 und 184 über zwei Leitungsäste 194 und 196 in die Leitung 30 zurück. An den Verzweigungspunkten ist jeweils eine Schalteinrichtung 200 und 202 vorgesehen mit der wechselweise die Leitungen 26 bzw. 30 mit einer der Leitungen 190 oder 192, bzw. 194 oder 196 verbunden werden können. Vorzugsweise sind die Schalbeinrichtungen gegenläufig geschaltet, d.h., daß beim Füllen des einen Zweiges der andere Zweig geleert werden kann und umgekehrt.

Als Schalteinrichtungen 200 und 202 können Drei-Wege Ventile, Doppelklemmen usw. in Frage kommen, die mit den Umsteuerungszeitpunkt der Pumpen 186 und 188 synchronisiert sind. So können diese Schalteinrichtungen entweder durch Sensoren umgeschaltet werden, die zugleich die Pumpen umschalten, oder aber zwangsläufig am Totpunkt der Hubkolbenpumpen geschaltet werden.

Weiterhin kann es vorteilhaft sein, daß die Pumpen 186 und 188 zu einer Pumpe zusammengefaßt sind, beispielsweise zu einer Hubkolbenpumpe, die mit einer Kolbenstange zwei Bälge gegenläufig betätigt.

Es muß nicht hinzugefügt werden, daß bei dem in Fig. 9 gezeigten Pumpensystem eine Druck- und Zwangssteuerung möglich ist.

Die Ventile oder Absperrorgane 128 und 132 können in einer weiteren Ausführungsform auch ohne elektrische Verbindung mit der Hubkolbenpumpe 124 allein durch eine mechanische Betätigung, beispielsweise durch nicht gezeigte Nocken, die auf dem Balg oder vorteilhafterweise auf dem Kolben der Hubkolbenpumpe angebracht sind, gesteuert werden. Durch eine entsprechende Verschiebbarkeit dieser Nocken kann der zweckmäßige Öffnungszeitpunkt und damit der Druckwert in der Leitung 122 eingestellt werden.

Besonders zu beachten ist weiterhin, daß bei Alarm, insbesondere beim Lösen der Förderleitung 26, neben dem Abschalten der wichtigsten Organe auch die Arterienklemme 31 geschlossen wird, so daß die vollständige Sicherheit des Patienten hergestellt ist.

**Patentansprüche**

1. Vorrichtung zum Reinigen des Blutes von Stoffwechselprodukten, insbesondere Single-Needle-Dialysevorrichtung, mit einem Dialysator (40), der einen Dialysierflüssigkeitsweg und einen Blutweg aufweist, wobei der Blutweg über eine Zuleitung (26) und eine Ableitung (30) mit wenigstens einem Blutanschluß (20) verbunden ist, die Zuleitung (26) eine Pumpe (34) aufweist, jeweils stromauf und stromab der Pumpe (34) Absperrorgane (31, 38, 42) vorgesehen sind und die Pumpe (34) ein im wesentlichen starres Gehäuse (68) und eine flexible, innerhalb des Gehäuses (68) angeordnete Membran aufweist, die den Innenraum des Gehäuses (68) in eine erste mit Blut beaufschlagbare Kammer (74) und eine zweite Kammer (78) trennt, wobei die zweite Kammer (78) über eine Leitung (82, 104, 116) mit einer als Antrieb für die Pumpe (34) dienenden Hubkolbenpumpe (36, 124) verbunden ist, die über die Leitung (82, 104, 116) ein Antriebsmedium in die zweite Kammer (78) hinein und wieder herauspumpt, dadurch gekennzeichnet, daß das Antriebsmedium ein Gas ist und der das Antriebsmedium enthaltende Raum mit einem Unterdruckventil (128), das sich in der Expansionsphase der Pumpe (34) bei einem vorbestimmten Unterdruck öffnet, und mit einem Überdruckventil (132) verbunden ist, das sich in der Kompressionsphase der Pumpe (34) bei einem vorbestimmten Überdruck öffnet.

2. Vorrichtung nach Anspruch 1, dadurch gekennzeichnet, daß das Hubvolumen der Hubkolbenpumpe (36, 124) größer ist als das Speichervolumen der Pumpe (34).

3. Vorrichtung nach Anspruch 2, dadurch gekennzeichnet, daß das Hubvolumen bis zu 20 % größer ist als das Speichervolumen.

4. Vorrichtung nach einem der Ansprüche 1 - 3, dadurch gekennzeichnet, daß in der die Pumpe (34) und die Hubkolbenpumpe (36, 124) verbindenden Leitung (116) eine hydrophobe, gasdurchlässige Membran (132) vorgesehen ist, die den Innenquerschnitt der Leitung (116) abdeckt.

5. Vorrichtung nach einem der Ansprüche 1 - 4, dadurch gekennzeichnet, daß die Hubkolbenpumpe (36, 124) zwangsgesteuert ist und an ihren Umkehrpunkten die Absperrorgane (31, 38, 42) umgeschaltet werden.

6. Vorrichtung nach einem der Ansprüche 1 - 5, dadurch gekennzeichnet, daß die Ventile (128, 132) und die Hubkolbenpumpe (36, 124) mit einer Überwachungseinrichtung (131) verbunden sind.

7. Vorrichtung nach Anspruch 6, dadurch gekennzeichnet, daß mit der Überwachungseinrichtung (131) die Pumpgeschwindigkeit gesteuert die zeitliche oder phasengebundene Abfolge der Ventilzustände registriert oder ein Alarmsignal abgegeben wird.

8. Vorrichtung nach einem der Ansprüche 1 - 7, dadurch gekennzeichnet, daß in der Pumpleitung (122) ein Drucksensor angeordnet ist, mit dem über die Uberwachungseinrichtung (131) die Änderung der Druckabfolge registriert wird.

9. Vorrichtung nach einem der Ansprüche 1 - 8, dadurch gekennzeichnet, daß die flexible Membran als Schlauch (66) ausgebildet ist.

## Claims

1. Device for clearing the blood from products of metabolism, in particular single-needle-dialysis system, comprising a dialyzer (40) which comprises a dialysate path and a blood path, whereby the blood path is connected to at least one blood connection (20) via a feed line (26) and a discharge line (30), whereby the feed line (26) comprises a pump (34), whereby shut-off members (31, 38, 42) are provided streamup and streamdown of the pump (34), respectively and the pump (34) comprises a substantially rigid housing (68) and a flexible membrane, positioned within said housing (68), said membrane dividing the interior of said housing (68) into a first chamber (74) loadable with blood and a second chamber (78), whereby the second chamber (78) is connected via a line (82, 104, 116) with a lifting piston pump (36, 124) serving as drive for the pump (34), said lifting piston pump pumping a drive medium via the line (82, 104, 116) into the second chamber (78) and out again, characterized in that the drive medium is a gas and the space containing the drive medium is connected with a vacuum valve (128) which opens in the expansion phase of the pump (34) at a predetermined negative pressure, and with a pressure relief valve (132) which opens in the compression phase of the pump (34) at a predetermined positive pressure.

2. Device according to claim 1, characterized in that the pump volume of the piston pump (36, 124) is greater than the storage volume of the pump (34).

3. Device according to claim 2, characterized in that the pump volume is up to 20 % greater than the storage volume.

4. Device according to one of claims 1 to 3, characterized in that in line (116) connecting the pump (34) and the piston pump (36, 124) a hydrophobic, gas-permeable membrane (132) is provided which covers the inner cross-section of the line (116).

5. Device according to one of claims 1 to 4, characterized in that the piston pump (36, 124) is forced driven and that at its reversal points the shut-off members (31, 38, 42) are switched over.

6. Device according to one of claims 1 to 5, characterized in that the valves (128, 132) and the piston pump (36, 124) are connected with a monitoring unit (131).

7. Device according to claim 6, characterized in that with the monitoring unit (131) the pump speed is controlled, the temporal or phase-bound sequence of the valve conditions registered or an alarm signal is given.

8. Device according to one of claims 1 to 7, characterized in that in the pump line (122) a pressure sensor is arranged with which via the monitoring unit (131) the change of the pressure is registered.

9. Device according to one of claims 1 to 8, characterized in that the flexible membrane is embodied as hose (66).

## Revendications

1. Dispositif de purification du sang par élimination de produits de métabolisme, en particulier dispositif de dialyse à une seule aiguille, comportant un dialyseur (40) qui présente un trajet pour le liquide de dialyse et un trajet pour le sang, dans lequel le trajet pour le sang est raccordé par l'intermédiaire d'une conduite d'alimentation (26) et d'une conduite d'évacuation (30) à au moins un raccord de prise de sang (20), la conduite d'alimentation (26) comporte une pompe (34), des organes d'arrêt (31, 38, 42) sont prévus tant en amont qu'en aval de la pompe (34) et la pompe (34) comporte un boîtier essentiellement rigide (68) et une membrane flexible disposée à l'intérieur du boîtier (68) et divisant l'intérieur de celui-ci en une première chambre (74) pouvant être alimentée en sang et une seconde chambre (78), la seconde chambre (78) étant raccordée par une conduite (82, 104, 116) à une pompe à piston alternatif (36, 124) servant de moyen d'entraînement pour la pompe (34), qui, par la conduite (82, 104, 116), introduit un agent d'entraînement dans la seconde chambre (78) et l'extrait à nouveau, caractérisé en ce que l'agent d'entraînement est un gaz et la chambre contenant l'agent d'entraînement est raccordée à une valve de dépression (128) qui s'ouvre dans la phase de détente de la pompe (34), à une dépression prédéterminée, et à une valve de surpression (132) qui s'ouvre dans la phase de compression de la pompe (34), à une surpression prédéterminée.

2. Dispositif suivant la revendication 1, caractérisé en ce que le volume balayé par le piston de la pompe à piston alternatif (36, 124) est supérieur au volume d'accumulation de la pompe (34).

3. Dispositif suivant la revendication 2, caractérisé en ce que le volume balayé par le piston est supérieur jusqu'à 20 % au volume d'accumulation.

4. Dispositif suivant l'une quelconque des revendications 1 à 3, caractérisé en ce que dans la conduite (116) reliant la pompe (34) et la pompe à piston alternatif (36, 124) est prévue une membrane (132) hydrophobe perméable aux gaz qui couvre la section intérieure de la conduite (116).

5. Dispositif suivant l'une quelconque des revendications 1 à 4, caractérisé en ce que la pompe à piston alternatif (36, 124) est commandée positivement et les organes d'arrêt (31, 38, 42) sont commutés à ses points de rebroussement.

6. Dispositif suivant l'une quelconque des revendications 1 à 5, caractérisé en ce que les valves (128, 132) et la pompe à piston alternatif (36, 124) sont reliées à un dispositif de surveillance (131).

7. Dispositif suivant la revendication 6, caractérisé en ce que le dispositif de surveillance (131) régit la vitesse de la pompe, enregistre la séquence chronologique ou liée aux phases des positions des valves ou produit un signal d'alarme.

8. Dispositif suivant l'une quelconque des revendications 1 à 7, caractérisé en ce qu'un capteur de pression est disposé dans la conduite de pompe (122) et permet par l'intermédiaire du dispositif de surveillance (131) d'enregistrer la modification de la séquence de pression.

9. Dispositif suivant l'une quelconque des revendications 1 à 8, caractérisé en ce que la membrane flexible a la forme d'un tuyau flexible (66).

Fig.1

Fig. 2

Fig. 3

Fig. 4

p (mmHg)

220 200 180 160 140 120 100 80 60 40 20

2 4 6 8 10 12 14 16 18 20 22 24 26 28

(ml)

Fig. 5

110
132
124
122
118
114
136
126
116
120
128
112
131

Fig. 6

26
52
162
62
46
68
160
94
66

Fig. 7

26
52
168
62
46
68
164
166
94
66

Fig. 8

26
52
168
62
46
68
166
170
94
66

Fig. 9